# EUROPEAN PATENT APPLICATION

(11) **EP 4 621 040 A2**
(43) Date of publication of application: **24.09.2025**
(21) Application number: 24209377.1
(22) Date of filing: 29.10.2024
(51) Int. Cl.: C12M 1/00, C12M 3/00

(54) **PHOTOSYNTHETIC BIOREACTOR SYSTEM**

(30) Priority: 21.03.2024 CN 202410329034
(71) Applicant: Alcarbo Technologies Limited, Pak Shek Kok., N.T. (HK)
(72) Inventor: POK HIM, NG, Mong Kok (HK); YAN CHU, CHEUNG, LAM TIN., KLN (HK); KWAN WAI, CHAN, Sheung Shui, NT (HK)
(74) Representative: Isern Patentes y Marcas S.L.

(57) **Abstract**

The embodiments of the present application relate to a photosynthetic bioreactor system in the field of bioreactors. The system includes at least one bioreactor with a transparent cultivation region for photosynthetic microorganisms; a conditioning device for producing ideal liquid culture medium; a collection device for recovering organic matter from the bioreactor; and a nano-bubble generating device for converting carbon dioxide-containing gas into minute bubbles for liquid introduction. The bioreactor includes a culture medium inlet, a culture medium outlet, and a gas inlet communicating with the transparent cultivation region. The conditioning device connects to each bioreactor's culture medium inlet, the collection device connects to each culture medium outlet, and the nano-bubble generating device connects to each air inlet. The transparent cultivation region has a thickness less than a preset dimension to enable light penetration.

## Description

### TECHNICAL FIELD

The present application relates to the field of bioreactor technology, and particularly relates to a photosynthetic bioreactor system.

### BACKGROUND

Photosynthetic bioreaction is a technology that utilizes light energy and microorganisms for photosynthesis. Its technical principle is utilizing light energy and photosynthetic microorganisms (such as algae, bacteria, or fungi) to conduct photosynthesis, converting carbon dioxide and sunlight into organic matter and oxygen.

Compared to traditional industrial production, it has many advantages. On one hand, it can utilize solar energy as a renewable energy source, reducing dependency on fossil fuels. On the other hand, it can absorb large amounts of carbon dioxide, helping to reduce greenhouse gas emissions. Furthermore, it has relatively high biomass productivity and growth rate, as well as lower land use requirements.

As environmental protection concepts continue to gain attention, this technology has begun to be widely applied in many fields, such as biofuel production, organic matter production, wastewater treatment, climate regulation, and biopharmaceutical production, and is playing an important role in sustainable development and environmental protection.

However, existing photosynthetic bioreactor systems still have many deficiencies in use, unable to well meet the needs of practical applications, and still need improvement in production and reaction efficiency. Therefore, there is an urgent need to provide suitable, more efficient photosynthetic bioreactor systems.

### SUMMARY

Embodiments of the present application are directed to providing a photosynthetic bioreactor system capable of addressing the deficiency of low production efficiency in existing bioreactors.

The embodiment of the present application discloses a photosynthetic bioreactor system. The photosynthetic bioreactor system includes: at least one bioreactor provided with a transparent cultivation area for photosynthetic microorganisms, configured to: enable the photosynthetic microorganisms to utilize light energy to convert carbon dioxide into organic matter; a conditioning device, configured to: produce ideal liquid culture medium; a collection device, configured to: recover organic matter formed by the bioreactor; a nanobubble generator device, configured to: convert gas containing carbon dioxide into tiny bubbles introduced into liquid; wherein the diameter of the tiny bubbles is between N* 10 nanometers to N* 100 nanometers, where N is a positive integer; wherein the bioreactor is provided with a culture medium inlet, a culture medium outlet and a gas inlet communicating with the transparent cultivation area; the conditioning device is connected to the culture medium inlet of each said bioreactor, the collection device is connected to the culture medium outlet of each bioreactor; the nanobubble generator device is connected to the air inlet of each bioreactor; the thickness of transparent cultivation area is less than a preset size, so that the light penetrates the transparent cultivation area.

Optionally, the bioreactor is a plate-like structure formed by two opposite end faces and wall faces connecting the two end faces;
wherein the spacing between two opposite end faces is the thickness of the transparent cultivation area; both end faces and wall faces of the bioreactor are made of transparent material.

Optionally, when said bioreactor is placed on the ground through supporting bracket, the projection area of bioreactor on the ground is significantly smaller than the projection area of the bioreactor in the direction perpendicular to the ground.

Optionally, the supporting bracket includes a fixed portion formed an accommodation space matching the thickness of the bioreactor, so that bioreactor is confined within the accommodation space; a supporting portion extended from the bottom end of the fixed portion along the direction parallel to the ground, forming several supporting feet; wherein the supporting feet have preset length, so that at least part of the gravity of the bioreactor confined in the accommodation space is transferred to the ground.

Optionally, the culture medium outlet is set on the bottom face of the bioreactor; the culture medium inlet and the air inlet are both set on the side wall of the bioreactor; wherein the bottom face of the bioreactor is the part of wall face closest to the ground when the bioreactor is placed on the ground through the supporting bracket; the side wall of the bioreactor is the part of wall face relatively perpendicular to the ground when the bioreactor is placed on the ground through the supporting bracket.

Optionally, the bioreactor has a slope inside the transparent cultivation area; wherein the slope has a preset gradient, inclining from the side wall of the bioreactor towards the bottom face of the bioreactor.

Optionally, the transparent material includes flexible plastic and composite film; wherein the bottom face of the bioreactor is provided with an air tube composed of the plastic film, and the air tube forms the air inlet of the bioreactor.

Optionally, the conditioning device is provided with sensors; a collection data of the sensors is fed back to a control unit, so that the liquid culture medium in an ideal state; wherein the sensors are selected from one or more of the following: dissolved carbon dioxide sensor, dissolved oxygen sensor, pH sensor, temperature sensor, turbidity sensor, dissolved solids sensor and fluorescence sensor.

Optionally, the photosynthetic microorganisms are selected from one or more of the following blue-green algae or microalgae:
*Nannochloropsis oculata; Nannochloropsis salina; Nannochloropsis sp.; Tetraselmis suecica; Tetraselmis chuii; Botryococcus braunii; Chlorella sp.; Chlorella ellipsoidea; Chlorella emersonii; Chlorella minutissima; Chlorella protothecoides; Chlorella pyrenoidosa; Chlorella salina; Chlorella sorokiniana; Chlorella vulgaris; Chroomonas salina; Cyclotella cryptica; Cyclotella sp.; Dunaliella salina; Dunaliella bardawil; Dunaliella tertiolecta; Euglena gracilis; Gymnodinium nelsoni; Haematococcus pluvialis; Isochrysis galbana; Monoraphidium minutum; Monoraphidium sp.; Nannochloris sp.; Neochloris oleoabundans; Nitzschia laevis; Onoraphidium sp.; Pavlova lutheri; Phaeodactylum tricornutum; Porphyridium cruentum; Scenedesmus obliquus; Scenedesmus quadricauda; Scenedesmus sp.; Skeletonema; Stichococcus bacillaris; Spirulina platensis; Thalassiosira sp.* the light includes sunlight and/or diffused light produced by lighting device; and the gas containing carbon dioxide includes: air, carbon dioxide gas, power plant exhaust gas or combustion chamber exhaust gas.

Optionally, the collection device includes: a tank body connected to the culture medium outlet of each bioreactor through a recovery pipeline, collecting organic matter flowing out with the liquid culture medium; a conical portion formed at the bottom of the tank body, configured to: separate the organic matter and the liquid culture medium; wherein the end of the conical portion forms organic matter outlet for outputting the separated organic matter; the tank body is connected to the conditioning device through a circulation pipeline for re-inputting the separated liquid culture medium into the conditioning device.

An advantageous aspect of the photosynthetic bioreactor system according to embodiments of the present application is that: through the incorporation of an additional nanobubble generator device, nanometer-scale bubbles having a significantly higher surface area-to-volume ratio compared to conventional bubbles can be produced, thereby effectively enhancing the solubility of carbon dioxide gas in the liquid medium and consequently improving the photosynthetic reaction efficiency of the photosynthetic bioreactor. Furthermore, through the design of an appropriate cultivation area thickness, penetration of external light through the entire cultivation area is ensured, thus providing optimal light conditions for algal photosynthesis and growth.

### BRIEF DESCRIPTION OF THE DRAWINGS

Exemplary illustrations of one or more embodiments are provided through corresponding figures in the accompanying drawings. These exemplary illustrations should not be construed as limiting the scope of the embodiments. Elements denoted by identical reference numerals in the drawings represent similar elements. Unless specifically stated otherwise, the drawings are not drawn to scale.
FIG. 1 illustrates a schematic view of a photosynthetic bioreactor system according to embodiments of the present application;
FIG. 2 illustrates a structural schematic view of a bioreactor according to embodiments of the present application;
FIG. 3 illustrates a structural schematic view of a supporting bracket according to embodiments of the present application;
FIG. 4 illustrates a structural schematic view of a collection device according to embodiments of the present application.

### DETAILED DESCRIPTION

To facilitate understanding of the present application, more detailed descriptions of the present application are provided below in conjunction with the drawings and specific embodiments. It should be noted that when an element is described as being "fixed to" another element, it can be directly on the other element, or one or more intermediate elements may exist between them. When an element is described as being "connected to" another element, it can be directly connected to the other element, or one or more intermediate elements may exist between them. The terms "upper," "lower," "inner," "outer," "bottom," etc. used in this specification indicating directional or positional relationships are based on the directional or positional relationships shown in the drawings, are only for the convenience of description and simplification of the present application, and do not indicate or imply that the referenced device or element must have a specific orientation, be constructed in a specific orientation, or operate in a specific orientation, therefore should not be construed as limitations of the present application. Furthermore, terms such as "first," "second," "third," etc. are used only for descriptive purposes and should not be construed as indicating or implying relative importance.

Unless otherwise defined, all technical and scientific terms used in this specification have the same meanings as commonly understood by those skilled in the art to which this application pertains. The terms used in the specification of the present application are only for the purpose of describing specific embodiments and are not intended to limit the present application. The term "and/or" used in this specification includes any and all combinations of one or more of the listed related items.

Moreover, the technical features involved in different embodiments of the present application described below can be combined with each other as long as they do not conflict with each other.

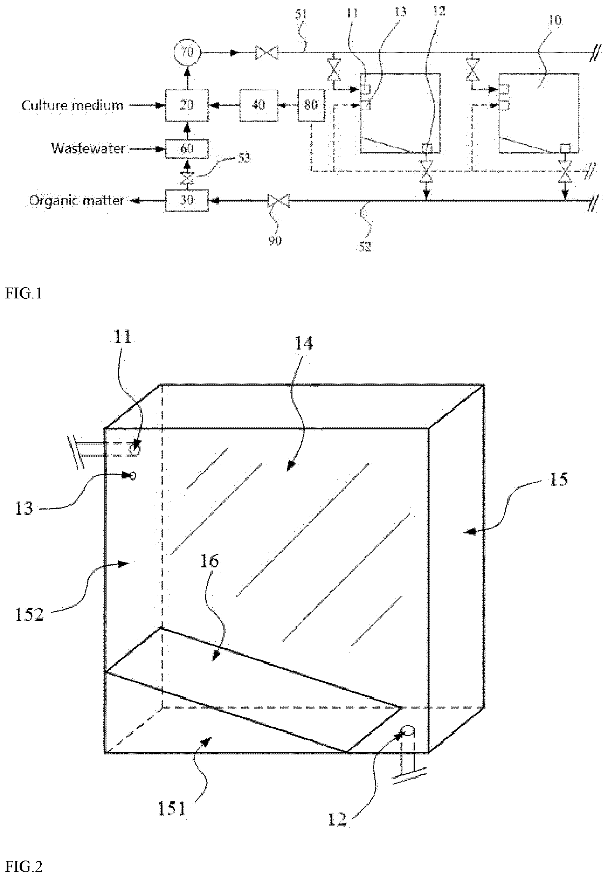

FIG. 1 is a schematic diagram of the photosynthetic bioreactor system according to embodiments of the present application. It shows the connection relationships between various functional modules in the system and the direction of material transfer. As shown in FIG. 1, the bioreactor system includes: at least one bioreactor 10, a conditioning device 20, a collection device 30, and a nanobubble generator device 40.

The bioreactor 10 is the basic element for photosynthesis in the system. It provides a transparent cultivation area for photosynthetic microorganisms, enabling them to utilize light energy to convert carbon dioxide into organic matter, thus achieving the aforementioned photosynthetic biological reaction.

Furthermore, the bioreactor 10 can have an appropriate structural design to ensure that the thickness of the transparent cultivation area is less than a preset dimension, thereby ensuring light can penetrate the entire transparent cultivation area, meeting the light energy requirements for algal photosynthesis.

Specifically, the number of bioreactors 10 can be set or adjusted according to actual conditions, with no specific limitations. For example, technicians can choose an appropriate number of bioreactors 10 based on actual usage requirements and limiting conditions (such as the amount of target carbon dioxide to be processed and the size of the installation site).

The conditioning device 20 is equipment used for processing and adjusting liquid materials. It can ensure the produced liquid culture medium meets intended use through one or more operations such as mixing, stirring, conditioning, and processing. In this embodiment, the term "ideal" is used to indicate that the liquid culture medium is in an ideal state that meets the intended use.

Preferably, the conditioning device 20 can also be equipped with one or more sensors to detect one or more current characteristics of the liquid culture medium. The data collected by these sensors can be fed back to a control unit, which after calculation and feedback, ensures the liquid culture medium in the conditioning device 20 maintains the ideal state through appropriate operational means. For example, by adding different culture medium materials or one or more regulators.

The sensors are selected from one or more of the following: dissolved carbon dioxide sensor, dissolved oxygen sensor, pH sensor, temperature sensor, turbidity sensor, dissolved solids sensor, and fluorescence sensor.

The collection device 30 is equipment used for recovering organic matter. It can be used to recover organic matter formed by the bioreactor and output it through specific output ports for subsequent steps. In this embodiment, the "organic matter" can be considered as the portion of algae after growth and proliferation.

The nanobubble generator device 40 is a bubble generator capable of generating nanometer-scale bubbles. It can introduce carbon dioxide-containing gas into the liquid and produce tiny bubbles using ultrasound, electrolysis, pressure waves, or other mechanisms.

Specifically, the diameter of these tiny bubbles can range between N* 10 nanometers to N* 100 nanometers, where N is a positive integer. In other words, these tiny bubbles can have bubble diameters of tens to hundreds of nanometers, thus providing good carbon dioxide solubility.

Please continue to refer to FIG. 1, in the complete bioreactor system, each bioreactor 10 is equipped with a culture medium inlet 11, a culture medium outlet 12, and a gas inlet 13, all communicating with the transparent cultivation area.

The conditioning device 20 can connect to the culture medium inlet 11 of each bioreactor 10 through liquid input pipeline 51, providing liquid culture medium to bioreactor 10 and controlling the culture medium level height inside bioreactor 10.

The collection device 30 connects to the culture medium outlet 12 of each bioreactor 10 through liquid recovery pipeline 52, recovering the organic matter produced by photosynthesis from bioreactor 10 through the recovery of liquid culture medium.

The nanobubble generator device 40 is introduced into the system, connecting to the air inlet 13 of each bioreactor, utilizing external gas sources to generate tiny bubbles introduced into the liquid culture medium, thereby providing sufficient carbon dioxide for photosynthesis.

The above system can also add one or more connection pipelines and/or functional modules according to actual needs to support different practical application scenarios. For example, a circulation pipeline 53 can be added between the collection device 30 and conditioning device 40, allowing the liquid culture medium recovered by the collection device 30 to be re-input into the conditioning device 20, achieving liquid culture medium recycling, or additional liquid pumps 70 or air pumps 80 can be added to the pipelines to help improve liquid/gas flow rates. Optionally, valves 90 can be added to one or more pipelines to control liquid flow rates or shut off pipelines to adapt to different operating conditions.

In some embodiments, continuing to refer to FIG. 1, the system can further be applied to wastewater treatment, by adding an additional filtering device 60. The filtering device 60 connects to the circulation pipeline 53 and can filter incoming sewage or wastewater, then introduce the filtered water into the conditioning device 20 as part of the liquid culture medium ingredients, allowing it to be further processed by the subsequent bioreactor 10.

In some embodiments, as shown in FIG. 2, the aforementioned bioreactor 10 can be a plate-like structure formed by two opposing end faces 14 and a continuous wall surface 15 connecting these end faces.

The "plate-like structure" refers to a relatively flat structural component where the radial dimension is significantly greater than the axial dimension. Both end faces and wall surfaces of the bioreactor are made of transparent materials, and the spacing between the two opposing end faces 14 defines the thickness of the transparent cultivation area.

FIG. 2 is a structural schematic diagram of the bioreactor according to embodiments of the present application. For ease of description, FIG. 2 exemplarily shows a roughly cubic shape. However, those skilled in the art will understand that other similar three-dimensional shapes can also be used.

In preferred embodiments, the above plate-like bioreactor can be placed "upright" on the ground using a supporting bracket 70. Upright placement refers to a positioning method where the bioreactor's ground projection area is significantly smaller than its projection area in the direction perpendicular to the ground.

The "significant" indicates a substantial difference between the two, representing at least a difference of 50% or more. For example, the bioreactor's end face can be positioned at an angle nearly perpendicular to the ground.

This placement method can provide a larger light-receiving area while occupying a smaller ground area, improving the system's ground area utilization efficiency.

Specifically, continuing to refer to FIG. 2, the culture medium outlet 12 can be located on the bottom surface 151 of the bioreactor 10, while the culture medium inlet 11 and air inlet 13 are both located on the side wall 152 of the bioreactor.

The bottom surface 151 refers to the part of the wall surface 15 closest to the ground when the bioreactor is placed on the ground using the supporting bracket. For example, as shown in FIG. 2, the bottom surface is one of the opposing wall surfaces in the width direction of the cubic bioreactor.

The side wall 152 refers to the parts of the wall surface of the bioreactor 10 that are perpendicular to the ground when the bioreactor is placed on the ground using the supporting bracket. For example, as shown in FIG. 2, the side wall is one of the opposing wall surfaces in the length direction of the cubic bioreactor.

Thus, the culture medium outlet located on the bottom surface can conveniently carry out precipitated organic matter along with a portion of liquid culture medium from the bioreactor to the collection device. The installation height of the culture medium inlet 11 can help control the liquid culture medium height inside the bioreactor. The air inlet 13 can be appropriately positioned at a lower height relative to the culture medium inlet 11, ensuring that the generated tiny bubbles are introduced into the liquid culture medium rather than being directly exposed in the bioreactor's air layer.

In preferred embodiments, continuing to refer to FIG. 2, an additional slope 16 can be installed inside the transparent cultivation area of the bioreactor 10.

The slope 16 is designed with a preset angle of inclination. It slopes from the bioreactor's side wall 152 toward the bioreactor's bottom surface 151, thereby helping to increase upward circulation, which aids in exposing algae more uniformly to light sources and carbon dioxide. Furthermore, an appropriate slope also helps improve the efficiency of emptying liquid culture medium and photosynthetic microorganisms from inside the bioreactor.

Specifically, the transparent materials mentioned above may include: flexible plastics and composite films. These are supported by a rigid supporting frame, thus forming the aforementioned transparent cultivation area inside. Correspondingly, the bioreactor's bottom surface is also equipped with air tubes made of plastic film, which form the bioreactor's air inlet.

Alternatively, the transparent materials can also directly use rigid transparent glass or acrylic in part to replace the rigid supporting frame.

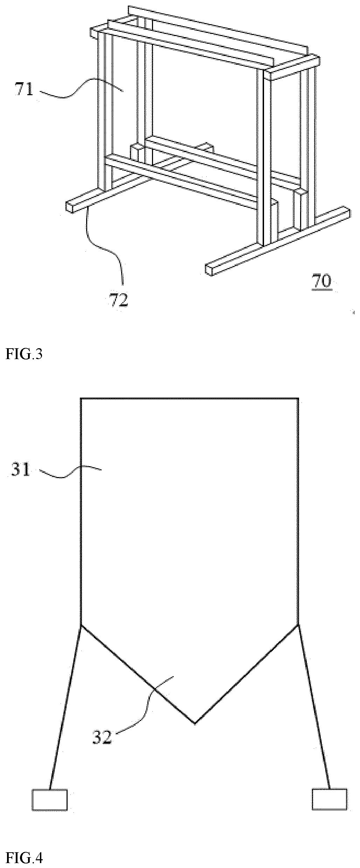

FIG. 3 is a schematic diagram of the supporting bracket according to embodiments of this application. This supporting bracket can be used in conjunction with the square bioreactor shown in FIG. 2, enabling it to be stably placed upright on the ground. As shown in FIG. 3, the supporting bracket 70 can include: a fixing portion 71 and a supporting portion 72.

The fixing portion 71 forms an accommodating space that matches the thickness of the bioreactor 10, thereby constraining the bioreactor 10 within this accommodating space.

The supporting portion 72 extends from the bottom of the fixing portion 71 parallel to the ground, forming several supporting legs. These legs have sufficient length to transfer at least part of the weight of the bioreactor constrained in the accommodating space to the ground.

Specifically, both the fixing portion 71 and supporting portion 72 can be manufactured through welding or other methods using metal tubes or strip-shaped metal materials. These metal tubes or strip-shaped surfaces can also be coated with anti-rust coating to enhance service life.

FIG. 4 is a schematic diagram of the collection device according to embodiments of this application. In some embodiments, as shown in FIG. 4, the collection device 30 includes: a tank body 31 and a conical portion 32.

The tank body 31 is the main part of the entire collection device. It can be roughly cylindrical in shape, connected to each bioreactor's culture medium outlet through recovery pipelines, receiving and storing the outflowing liquid culture medium.

The conical portion 32, formed at the bottom of the tank body 31, is a separation structure used to separate organic matter from the liquid culture medium. Using a steep slope, it can precipitate the organic matter that flows out with the liquid culture medium to the end of the conical portion 32, thereby achieving separation of organic matter from liquid culture medium.

During practical use, the separated organic matter is output through the organic matter outlet formed at the end of the conical portion 32. The separated liquid culture medium can then be returned to the conditioning device through circulation pipelines between the tank body 32 and the conditioning device. After being restored to ideal liquid culture medium conditions, it continues to be supplied to the bioreactor for use.

In some embodiments, the photosynthetic microorganisms are selected from one or more of the following blue-green algae or microalgae:

*Nannochloropsis oculata; Nannochloropsis salina; Nannochloropsis sp.; Tetraselmis suecica; Tetraselmis chuii; Botryococcus braunii; Chlorella sp.; Chlorella ellipsoidea; Chlorella emersonii; Chlorella minutissima; Chlorella protothecoides; Chlorella pyrenoidosa; Chlorella salina; Chlorella sorokiniana; Chlorella vulgaris; Chroomonas salina; Cyclotella cryptica; Cyclotella sp.; Dunaliella salina; Dunaliella bardawil; Dunaliella tertiolecta; Euglena gracilis; Gymnodinium nelsoni; Haematococcus pluvialis; Isochrysis galbana; Monoraphidium minutum; Monoraphidium sp.; Nannochloris sp.; Neochloris oleoabundans; Nitzschia laevis; Onoraphidium sp.; Pavlova lutheri; Phaeodactylum tricornutum; Porphyridium cruentum; Scenedesmus obliquus; Scenedesmus quadricauda; Scenedesmus sp.; Skeletonema; Stichococcus bacillaris; Spirulina platensis; Thalassiosira sp.*

Preferably, the light provided to the bioreactor in the system can be sunlight and diffused light generated by lighting devices. The diffused light is used to improve the photosynthetic efficiency of the bioreactor.

Specifically, the carbon dioxide-containing gas sources used in the system includes: air, carbon dioxide gas, power plant exhaust gas, or combustion chamber exhaust gas, depending on the actual application scenario. These carbon dioxide-containing gas sources are connected to the nano-bubble generator, so that when introduced into the liquid culture medium, they produce nano-sized bubbles.

It should be finally noted that the above-described embodiments are merely illustrative of the technical solutions of the present application and should not be construed as limiting thereof. Under the technical teaching of the present application, combinations of technical features between the above embodiments or different embodiments, and implementation of steps in any sequence are possible. While numerous other variations of different aspects of the present application exist as described above, they are not detailed herein for the sake of conciseness. Although the present application has been described in detail with reference to the foregoing embodiments, those of ordinary skill in the art should understand that: they may still make modifications to the technical solutions described in the foregoing embodiments, or make equivalent substitutions to some technical features thereof; and such modifications or substitutions shall not cause the essence of the corresponding technical solutions to depart from the scope of the technical solutions of the various embodiments of the present application.

## Claims

1. A photosynthetic bioreactor system, comprising:
at least one bioreactor provided with a transparent cultivation area for photosynthetic microorganisms, configured to: enable the photosynthetic microorganisms to utilize light energy to convert carbon dioxide into organic matter;
a conditioning device, configured to: produce ideal liquid culture medium;
a collection device, configured to: recover organic matter formed by the bioreactor;
a nanobubble generator device, configured to: convert gas containing carbon dioxide into tiny bubbles introduced into liquid; wherein the diameter of the tiny bubbles is between N* 10 nanometers to N* 100 nanometers, where N is a positive integer;
wherein the bioreactor is provided with a culture medium inlet, a culture medium outlet and a gas inlet communicating with the transparent cultivation area;
the conditioning device is connected to the culture medium inlet of each said bioreactor,
the collection device is connected to the culture medium outlet of each bioreactor; the nanobubble generator device is connected to the air inlet of each bioreactor;
the thickness of transparent cultivation area is less than a preset size, so that the light penetrates the transparent cultivation area.

2. The photosynthetic bioreactor system according to claim 1, wherein the bioreactor is a plate-like structure formed by two opposite end faces and wall faces connecting the two end faces;
wherein the spacing between two opposite end faces is the thickness of the transparent cultivation area; both end faces and wall faces of the bioreactor are made of transparent material.

3. The photosynthetic bioreactor system according to claim 2, wherein when the bioreactor is placed on the ground through supporting bracket, the projection area of bioreactor on the ground is significantly smaller than the projection area of the bioreactor in the direction perpendicular to the ground.

4. The photosynthetic bioreactor system according to claim 3, wherein the supporting bracket comprises:
a fixed portion formed an accommodation space matching the thickness of the bioreactor, so that bioreactor is confined within the accommodation space;
a supporting portion extended from the bottom end of the fixed portion along the direction parallel to the ground, forming several supporting feet;
wherein the supporting feet have preset length, so that at least part of the gravity of the bioreactor confined in the accommodation space is transferred to the ground.

5. The photosynthetic bioreactor system according to claim 3, wherein the culture medium outlet is set on the bottom face of the bioreactor; the culture medium inlet and the air inlet are both set on the side wall of the bioreactor;
wherein the bottom face of the bioreactor is the part of wall face closest to the ground when the bioreactor is placed on the ground through the supporting bracket;
the side wall of the bioreactor is the part of wall face relatively perpendicular to the ground when the bioreactor is placed on the ground through the supporting bracket.

6. The photosynthetic bioreactor system according to claim 5, wherein the bioreactor has a slope inside the transparent cultivation area; wherein the slope has a preset angle of inclination, inclining from the side wall of the bioreactor towards the bottom face of the bioreactor.

7. The photosynthetic bioreactor system according to claim 5, wherein the transparent material comprises: flexible plastic and composite film;
wherein the bottom face of the bioreactor is provided with an air tube composed of the plastic film, and the air tube forms the air inlet of the bioreactor.

8. The photosynthetic bioreactor system according to claim 1, wherein the conditioning device is provided with sensors; a collection data of the sensors is fed back to a control unit, so that the liquid culture medium in an ideal state;
wherein the sensors are selected from one or more of the following: dissolved carbon dioxide sensor, dissolved oxygen sensor, pH sensor, temperature sensor, turbidity sensor, dissolved solids sensor and fluorescence sensor.

9. The photosynthetic bioreactor system according to claim 1, wherein the photosynthetic microorganisms are selected from one or more of the following blue-green algae or microalgae:
*Nannochloropsis oculata; Nannochloropsis salina; Nannochloropsis sp.; Tetraselmis suecica; Tetraselmis chuii; Botryococcus braunii; Chlorella sp.; Chlorella ellipsoidea; Chlorella emersonii; Chlorella minutissima; Chlorella protothecoides; Chlorella pyrenoidosa; Chlorella salina; Chlorella sorokiniana; Chlorella vulgaris; Chroomonas salina; Cyclotella cryptica; Cyclotella sp.; Dunaliella salina; Dunaliella bardawil; Dunaliella tertiolecta; Euglena gracilis; Gymnodinium nelsoni; Haematococcuspluvialis; Isochrysis galbana; Monoraphidium minutum; Monoraphidium sp.; Nannochloris sp.; Neochloris oleoabundans; Nitzschia laevis; Onoraphidium sp.; Pavlova lutheri; Phaeodactylum tricornutum; Porphyridium cruentum; Scenedesmus obliquus; Scenedesmus quadricauda; Scenedesmus sp.; Skeletonema; Stichococcus bacillaris; Spirulina platensis; Thalassiosira sp.* wherein the light comprises: sunlight and/or diffused light produced by lighting device;
the gas containing carbon dioxide comprises: air, carbon dioxide gas, power plant exhaust gas or combustion chamber exhaust gas.

10. The photosynthetic bioreactor system according to claim 1, wherein the collection device comprises:
a tank body connected to the culture medium outlet of each bioreactor through a recovery pipeline, collecting organic matter flowing out with the liquid culture medium;
a conical portion formed at the bottom of the tank body, configured to: separate the organic matter and the liquid culture medium;
wherein the end of the conical portion forms organic matter outlet for outputting the separated organic matter; the tank body is connected to the conditioning device through a circulation pipeline for re-inputting the separated liquid culture medium into the conditioning device.
